# EUROPÄISCHE PATENTANMELDUNG

(11) **EP 2 292 616 A1**
(43) Veröffentlichungstag der Anmeldung: **09.03.2011**
(21) Anmeldenummer: 10177691.2
(22) Anmeldetag: 22.09.2006
(51) Int. Cl.: C07D 413/14, A61K 31/5377, A61P 7/02

(54) **Neue polymorphe Form und die amorphe Form von 5-Chlor-N-({(5S)-2-oxo-3-[4-(3-oxo-4-morpholinyl)-phenyl]-1,3-oxazolidin-5-yl}-methyl)-2-thiophencarboxamid**

(30) Priorität: 04.10.2005 DE 102005047564
(62) Teilanmeldung aus: 06792210.4
(71) Anmelder: Bayer Schering Pharma Aktiengesellschaft, 13353 Berlin (DE)
(72) Erfinder: Grunenberg, Alfons, 41539, Dormagen (DE); Lenz, Jana, 42117, Wuppertal (DE); Braun, Gerhard, 50733, Köln (DE); Keil, Birgit, 40231, Düsseldorf (DE); Thomas, Christian, 42111, Wuppertal (DE)

(57) **Zusammenfassung**

Die vorliegende Erfindung betrifft eine neue polymorphc Form und die amorphe Form von 5-Chlor-N-({(5*S*)-2-oxo-3-[4-(3-oxo-4-morpholinyl)-phenyl]-1,3-oxazolidin-5-yl}-methyl)-2-thiophen-carboxamid, Verfahren zu deren Herstellung, diese enthaltende Arzneimittel sowie deren Verwendung bei der Bekämpfung von Krankheiten.

## Beschreibung

Die vorliegende Erfindung betrifft eine neue polymorphe Form und die amorphe Form von 5-Chlor-N-({(5*S*)-2-oxo-3-[4-(3-oxo-4-morpholinyl)-phenyl]-1,3-oxazolidin-5-yl}-methyl)-2-thiophen-carboxamid, Verfahren zu deren Herstellung, diese enthaltende Arzneimittel sowie deren Verwendung bei der Bekämpfung von Krankheiten.

Die Verbindung 5-Chlor-N-({(5*S*)-2-oxo-3-[4-(3-oxo-4-morpholinyl)-phenyl]-1,3-oxazolidin-5-yl}-methyl)-2-thiophencarboxamid ist aus WO 01/47949 und WO 2004/060887 bekannt und entspricht der Formel (I):

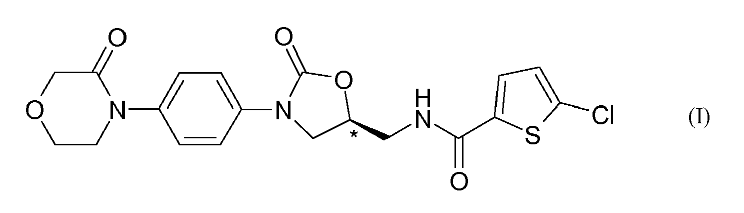

Die Verbindung der Formel (I) ist ein niedermolekularer, oral applizierbarer Inhibitor des Blutgerinnungsfaktors Xa, der zur Prophylaxe, Sekundärprophylaxe und/oder Behandlung von verschiedenen thromboembolischen Erkrankungen eingesetzt werden kann (siehe hierzu WO 01/47919, deren Offenbarung hiermit durch Bezugnahme eingeschlossen ist), insbesondere von Herzinfakt, Angina Pectoris (eingeschlossen instabile Angina), Reokklusionen und Restenosen nach einer Angioplastie oder aortokoronarem Bypass, Hirnschlag, transitorischen ischämischen Attacken, peripheren arteriellen Verschlusskrankheiten, Lungenembolien oder tiefen venösen Thrombosen.

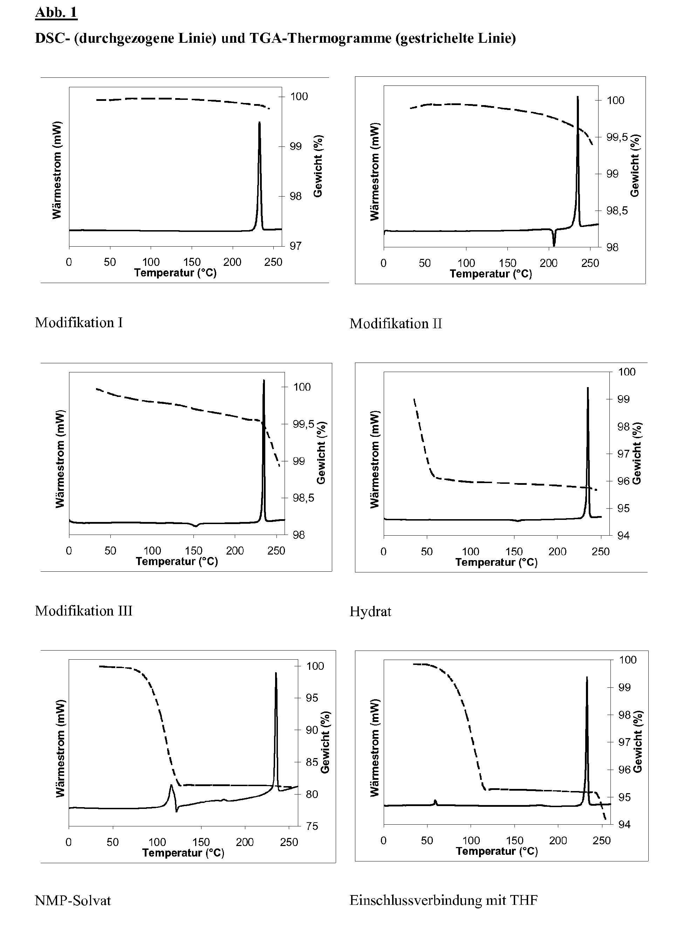

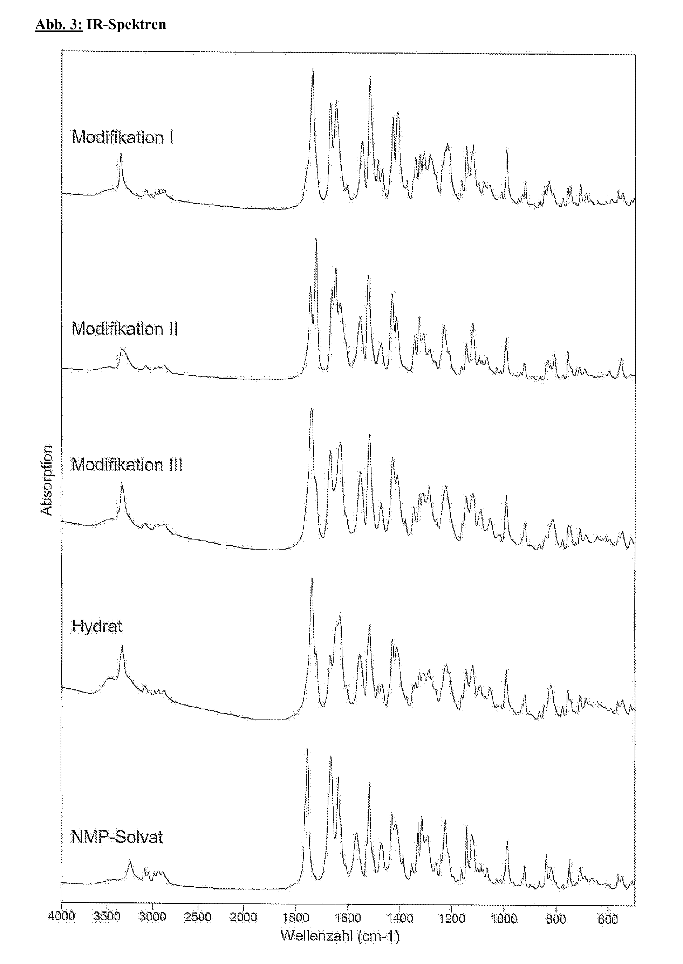

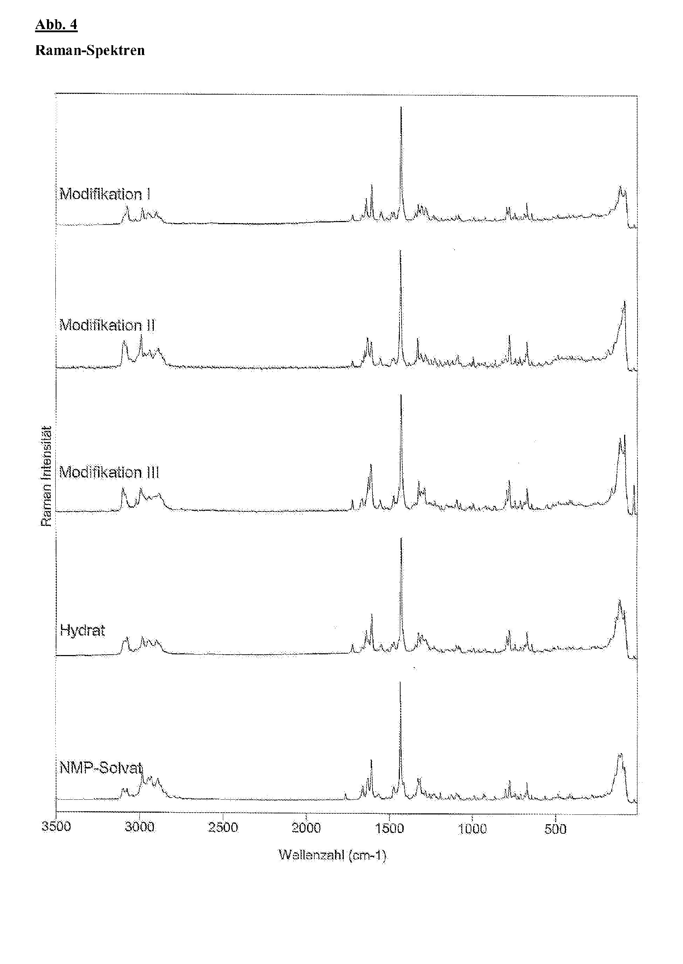

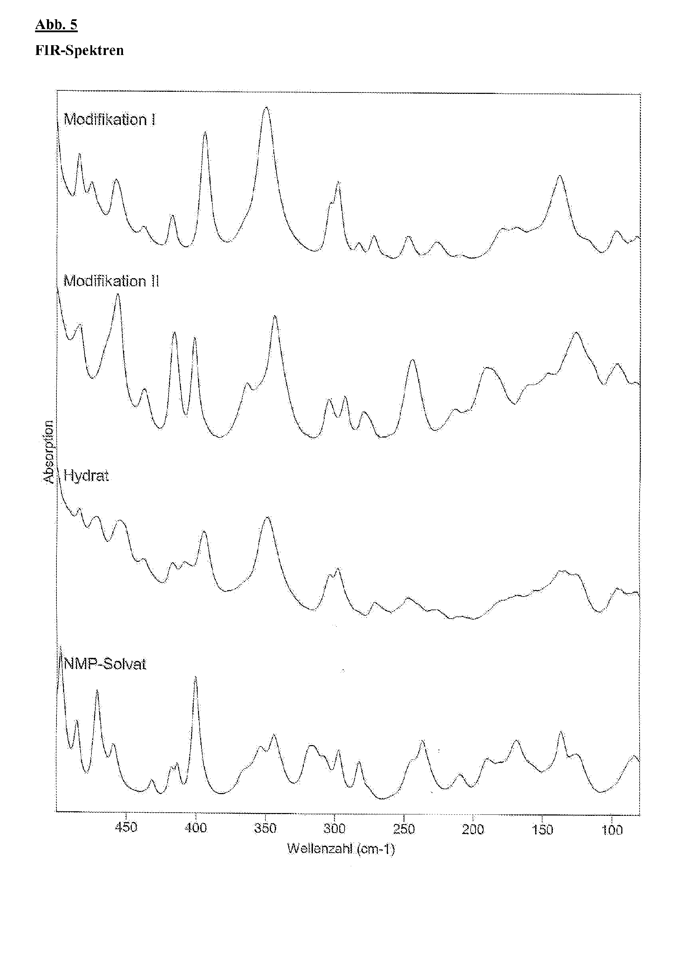

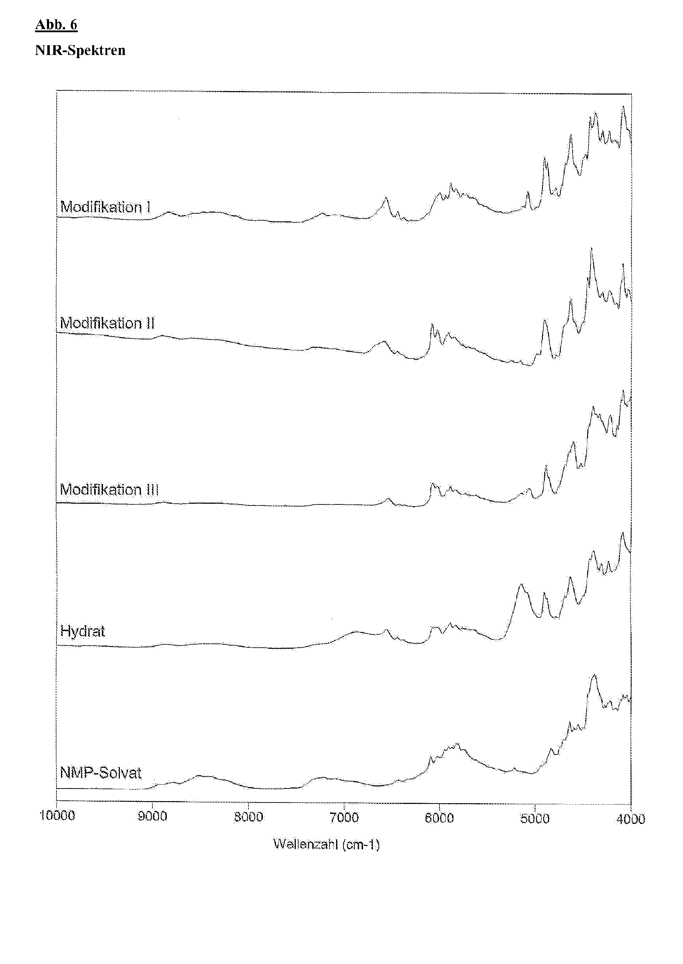

Die Verbindung der Formel (I) lässt sich wie in WO 01/47949 und WO 2004/060887 beschrieben herstellen. Dabei wird die Verbindung der Formel (I) in einer Kristallmodifikation erhalten, die im Folgenden als Modifikation I bezeichnet wird. Modifikation I hat einen Schmelzpunkt von 230°C und ein charakteristisches Röntgendiffraktogramm, IR-Spektrum, Raman-Spektrum, FIR-Spektrum und NIR-Spektrum (Tab. 1-6, Abb. 1-6). Es wurde nun gefunden, dass Modifikation I im Vergleich zur Modifikation II eine um den Faktor 4 geringere Löslichkeit aufweist.

Überraschenderweise wurden zwei weitere Modifikationen, ein Hydrat, ein NMP-Solvat und eine Einschlussverbindung mit THF der Verbindung der Formel (I) gefunden. Die Verbindung der Formel (I) in der Modifikation II schmilzt bei etwa 203 °C bzw. hat einen Umwandlungspunkt von etwa 195°C, die Verbindung der Formel (I) in der Modifikation III hat einen Umwandlungspunkt von etwa 127°C. Das Hydrat enthält etwa 4 % Wasser, das NMP-Solvat enthält 18,5 % N-Methylpyrrolidon und die Einschlussverbindung mit THF etwa 5-7 % Tetrahydrofuran.

Gegenstand der vorliegenden Erfindung ist die Verbindung der Formel (I) in der Modifikation II. Durch den erfindungsgemäßen Einsatz der Verbindung der Formel (I) in der Modifikation II wird sichergestellt, dass eine im Vergleich zur bekannten Modifikation höhere Löslichkeit erreicht wird.

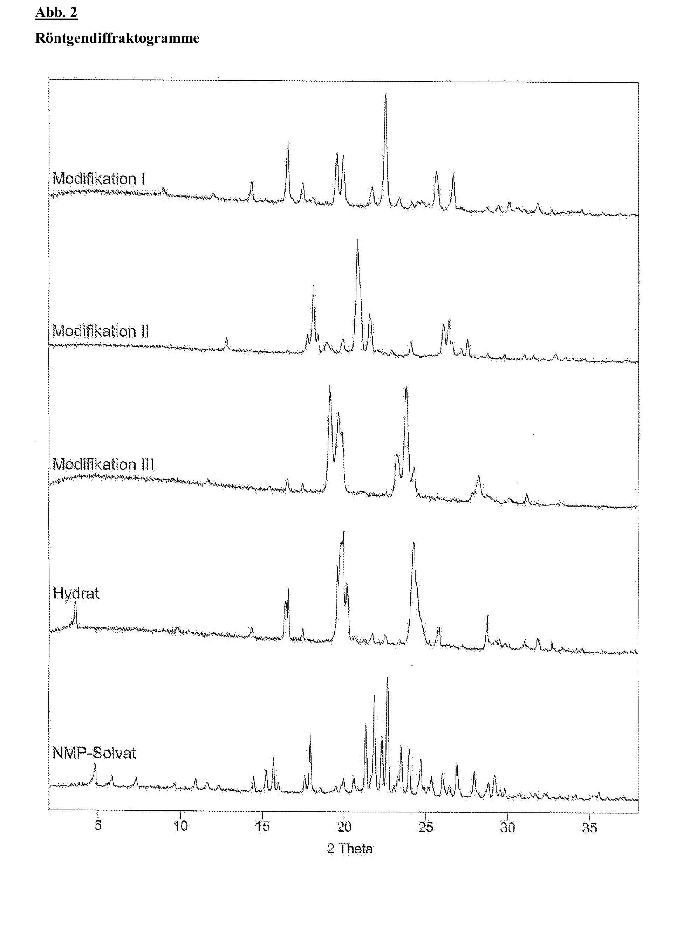

Modifikation II der Verbindung der Formel (I) hat im Vergleich zu Modifikation I, Modifikation III, der Hydratform, dem NMP-Solvat und der Einschlussverbindung mit THF ein klar unterscheidbares Röntgendiffraktogramm, IR-Spektrum, NIR-Spektrum, FIR-Spektrum und Raman-Spektrum (Abb. 2-6). Die Verbindung der Formel (I) in der Modifikation II schmilzt bei 203°C bzw. wandelt sich bei etwa 195°C um und ist damit klar unterscheidbar von Modifikation I (Schmelzpunkt 230°C) und Modifikation III (Umwandlungspunkt etwa 127°C). Im Gegensatz zu diesen lösungsmittelfreien Formen weisen das Hydrat der Verbindung der Formel (I), das NMP-Solvat der Verbindung der Formel (I) und die Einschlussverbindung mit THF der Verbindung der Formel (I) Masseverluste bei der thermogravimetrischen Analyse (TGA) von 4 %, 18,5 % bzw. 5-7 % auf (Abb. 1).

Es ist allgemein bekannt, dass kristalline polymorphe Formen eine schlechtere Wasserlöslichkeit aufweisen als die amorphe Form. Dies führt zu einer geringeren Bioverfügbarkeit im Vergleich zur amorphen Form.

Gegenstand der vorliegenden Erfindung ist weiterhin die Verbindung der Formel (I) in amorpher Form. Durch den erfindungsgemäßen Einsatz der Verbindung der Formel (I) in der amorphen Form wird sichergestellt, dass eine maximale Bioverfügbarkeit erreicht wird.

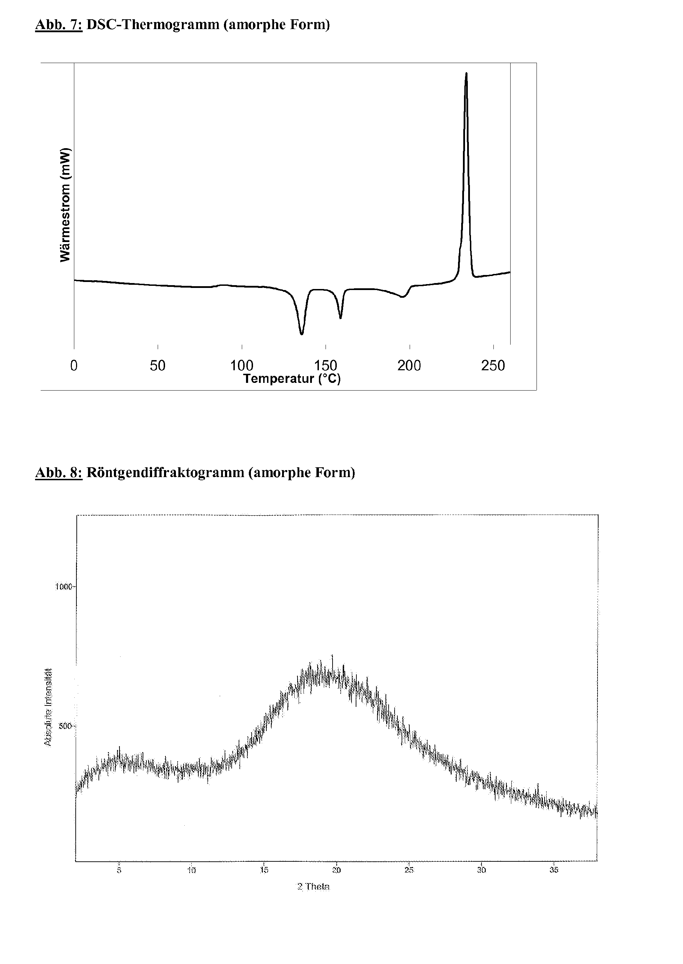

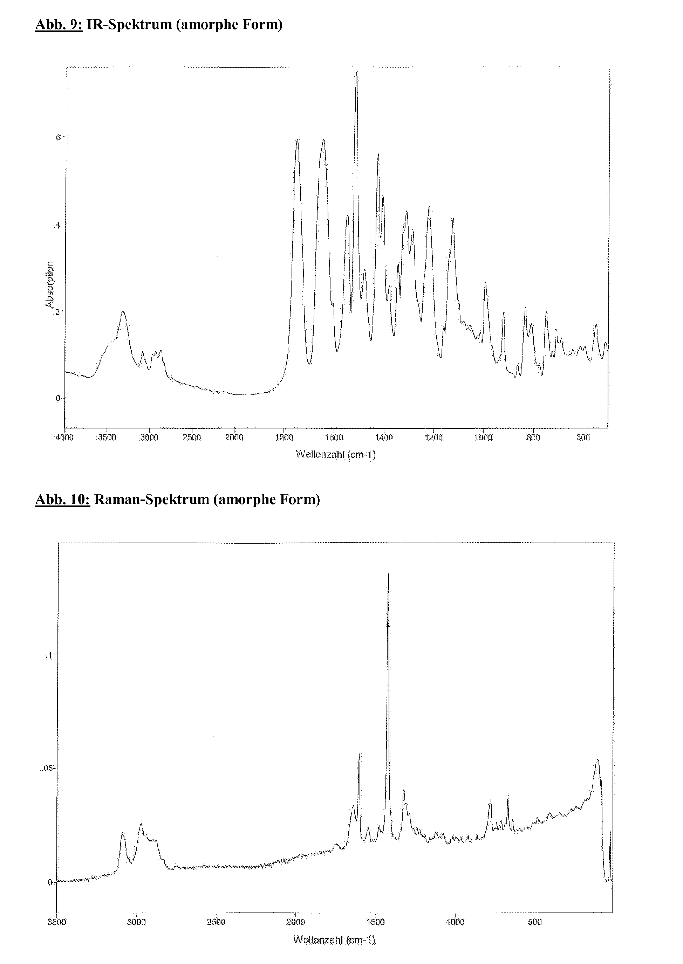

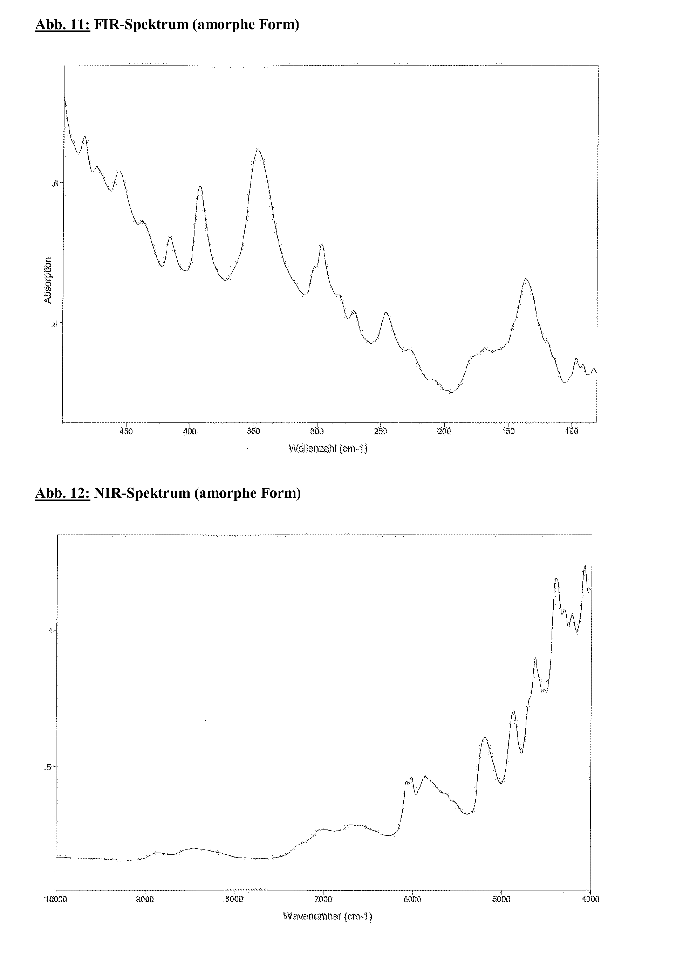

Die amorphe Form der Verbindung der Formel (I) hat ein charakteristisches Röntgendiffraktogramm, NIR-Spektrum, FIR-Spektrum und Raman-Spektrum (Abb. 8-12). Die Verbindung der Formel (I) in der amorphen Form hat eine Glasumwandlungstemperatur von etwa 83°C (DSC, Abb. 7).

Die erfindungsgemäße Verbindung der Formel (I) in der Modifikation II oder in der amorphen Form wird in pharmazeutischen Formulierungen in hoher Reinheit eingesetzt. Aus Stabilitätsgründen enthält eine pharmazeutische Formulierung hauptsächlich die Verbindung der Formel (I) in der Modifikation II oder in der amorphen Form und keine größeren Anteile einer anderen Form wie beispielsweise einer anderen Modifikation oder eines Solvates der Verbindung der Formel (I). Bevorzugt enthält das Arzneimittel mehr als 90 Gewichtsprozente, besonders bevorzugt mehr als 95 Gewichtsprozente der Verbindung der Formel (I) in der Modifikation II oder in der amorphen Form bezogen auf die Gesamtmenge der enthaltenen Verbindung der Formel (I). Weiterer Gegenstand der vorliegenden Erfindung ist der Einsatz der Verbindung der Formel (I) in der Modifikation II oder in der amorphen Form zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die erfindungsgemäße Verbindung eignet sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen. Darüber hinaus ist die erfindungsgemäße Verbindung zur Behandlung der disseminierten intravasalen Gerinnung (DIC) geeignet.

Thromboembolische Komplikationen treten ferner auf bei mikroangiopathischen hämolytischen Anämien, extrakorporalen Blutkreisläufen, wie Hämodialyse, sowie Herzklappenprothesen.

Außerdem kommt die erfindungsgemäße Verbindung auch für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem kann die erfindungsgemäße Verbindung zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Die erfindungsgemäße Verbindung kann darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor Xa enthalten.

Weiterer Gegenstand der vorliegenden Erfindung ist die Verwendung der erfindungsgemäßen Verbindung zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindung zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer antikoagulatorisch wirksamen Menge der erfindungsgemäßen Verbindung.

Weiterer Gegenstand der vorliegenden Erfindung ist ein Verfahren zur Verhinderung der Blutkoagulation *in vitro,* insbesondere bei Blutkonserven oder biologischen Proben, die Faktor Xa enthalten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend die erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren;
- Koronartherapeutika/Vasodilatatoren, insbesondere ACE-(Angiotensin-Converting-Enzyme)-Inhibitoren; AII-(Angiotensin II)-Rezeptor-Antagonisten; β-Adrenozeptor-Antagonisten; alpha-1-Adrenozeptor-Antagonisten; Diuretika; Calciumkanal-Blocker; Substanzen, die eine Erhöhung von cyclischem Guanosinmonophosphat (cGMP) bewirken, wie beispielsweise Stimulatoren der löslichen Guanylatcyclase;
- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren);
- antikoagulatorisch wirksame Substanzen (Antikoagulantien);
- plättchenaggregationshemmende Substanzen (Plättchenaggregationshemmer, Thrombozytenaggregationshemmer);
- sowie Fibrinogen-Rezeptor-Antagonisten (Glycoprotein-IIb/IIIa-Antagonisten).

Weiterer Gegenstand der vorliegenden Erfindung sind Arzneimittel, die die erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäße Verbindung kann systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege kann die erfindungsgemäße Verbindung in geeigneten Applikationsformen verabreicht werden.

Für die orale Applikation eignen sich nach dem Stand der Technik funktionierende, die erfindungsgemäß Verbindung schnell und/oder modifiziert abgebende Applikationsformen, die die Verbindung der Formel (I) in der Modifikation II oder in der amorphen Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Suspensionen oder Aerosole.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Suspensionen, Lyophilisaten oder sterilen Pulvern.

Für die sonstigen Applikationswege eignen sich z.B. Inhalationsarzneiformen (u.a. Pulverinhalatoren, Nebulizer), lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäße Verbindung kann in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Weiterer Gegenstand der Erfindung ist ein Verfahren zur Herstellung der Verbindung der Formel (I) in der Modifikation II, indem die Verbindung der Formel (I) in der Modifikation I in einem inerten Lösungsmittel gelöst und der Wirkstoff durch Zugabe eines Fällungsmittels bei einer Temperatur zwischen 0°C und 80°C, bevorzugt von 20 bis 25°C, gefällt wird. Der Niederschlag wird isoliert und getrocknet. Man erhält so die Verbindung der Formel (I) in der Modifikation II.

Ebenfalls Gegenstand der Erfindung ist ein Verfahren zur Herstellung der Verbindung der Formel (I) in der Modifikation II, indem die Verbindung der Formel (I) in der Modifikation I in einem inerten Lösungsmittel gelöst und, bevorzugt bei erhöhter Temperatur, insbesondere bei einer Temperatur von 30°C bis zur Rückflusstemperatur des Lösungsmittels, bis zum vollständigen Verdampfen des Lösungsmittels und Auskristallisieren des Wirkstoffs gelagert wird. Man erhält so die Verbindung der Formel (I) in der Modifikation II.

Ebenfalls Gegenstand der Erfindung ist ein Verfahren zur Herstellung der Verbindung der Formel (I) in der Modifikation II, indem die Verbindung der Formel (I) in der amorphen Form in einem wasserfreien inerten Lösungsmittel suspendiert und bis zum Erreichen des gewünschten Umwandlungsgrads, insbesondere bis zur quantitativen Umwandlung, in die Modifikation II gerührt oder geschüttelt wird. Das erhaltene Kristallisat wird isoliert und getrocknet. Man erhält so die Verbindung der Formel (I) in der Modifikation II.

Als inerte Lösungsmittel eignen sich niedere Alkohole wie beispielsweise Methanol, Ethanol, n-Propanol, iso-Propanol, n-Butanol, sec-Butanol, iso-Butanol, 1-Pentanol, oder Ketone wie Aceton, oder Alkane wie n-Pentan, Cyclopentan, n-Hexan, Cyclohexan, oder Tetrahydrofuran, oder Acetonitril, oder Toluol, oder Ethylacetat, oder 1,4-Dioxan, oder Gemische der genannten Lösungsmittel, oder Gemische der genannten Lösungsmittel mit Wasser. Bevorzugt sind Aceton, Tetrahydrofuran, 1-Pentanol oder Gemische der genannten Lösungsmittel. Als Fällungsmittel eignen sich inerte, wasserfreie Lösungsmittel, in denen der Wirkstoff schwer löslich ist, wie z. B. n-Heptan, Cyclohexan oder Toluol. Bevorzugt ist n-Heptan.

Bevorzugt wird die Verbindung der Formel (I) in der Modifikation II hergestellt, indem die Verbindung der Formel (I) in der Modifikation I in Aceton oder Tetrahydrofuran gelöst und der Wirkstoff durch Zugabe von n-Heptan bei einer Temperatur zwischen 0 und 80°C, bevorzugt bei einer Temperatur von 20 bis 25°C, gefällt wird. Der Niederschlag wird isoliert und getrocknet. Man erhält so die Verbindung der Formel (I) in der Modifikation II.

Ebenfalls bevorzugt wird die Verbindung der Formel (I) in der Modifikation II hergestellt, indem die Verbindung der Formel (I) in der Modifikation I in 1,4-Dioxan gelöst wird und bei erhöhter Temperatur, insbesondere bei einer Temperatur von 30°C bis zur Rückflusstemperatur des Lösungsmittels, beispielsweise 50°C, bis zum vollständigen Verdampfen des Lösungsmittels und Auskristallisieren des Wirkstoffs gelagert wird. Man erhält so die Verbindung der Formel (I) in der Modifikation II.

Ebenfalls bevorzugt wird die Verbindung der Formel (I) in der Modifikation II hergestellt, indem die Verbindung der Formel (I) in der amorphen Form in einem inerten wasserfreien Lösungsmittel suspendiert und bis zum Erreichen des gewünschten Umwandlungsgrads in die Modifikation II bei einer Temperatur von 20 bis 25°C gerührt oder geschüttelt wird. Das erhaltene Kristallisat wird isoliert und getrocknet Man erhält so die Verbindung der Formel (I) in der Modifikation II.

Weiterer Gegenstand der Erfindung ist ein Verfahren zur Herstellung der Verbindung der Formel (I) in der amorphen Form, in dem die Verbindung der Formel (I) in einer kristallinen Form durchgeschmolzen und anschließend schnell abgekühlt wird. Man erhält so die Verbindung der Formel (I) in der amorphen Form.

Bevorzugt wird die Verbindung der Formel (I) in der amorphen Form hergestellt, indem die Verbindung der Formel (I) in einer kristallinen Form bei einer Temperatur von mindestens 230°C, insbesondere bei einer Temperatur von 240 bis 250°C, durchgeschmolzen und anschließend schnell abgekühlt wird. Man erhält so die Verbindung der Formel (I) in der amorphen Form.

Von den kristallinen Formen Modifikation I, II und III werden dabei vorzugsweise Modifikation I oder II eingesetzt, insbesondere Modifikation I.

Durch das schnelle Abkühlen wird die Temperatur der Verbindung (I) vorzugsweise auf oder in die Nähe der Raumtemperatur gebracht, beispielsweise auf eine Temperatur von etwa 15 bis 30°C, insbesondere von etwa 20 bis 25°C. Das schnelle Abkühlen erfolgt vorzugsweise innerhalb weniger Sekunden, beispielsweise innerhalb von etwa 5 Sekunden. Zum schnellen Abkühlen wird vorzugsweise Schockkühlung eingesetzt.

Die Verbindung der Formel (I) in der Modifikation III kann hergestellt werden, indem die Verbindung der Formel (I) in der Modifikation I in einem inerten Lösungsmittel, beispielsweise Aceton, gelöst wird. Die Lösung wird mit Wasser versetzt und bei Raumtemperatur stehengelassen, bis das Lösungsmittel vollständig verdunstet ist. Man erhält so die Verbindung der Formel (I) in der Modifikation III.

Das Hydrat der Verbindung der Formel (I) kann hergestellt werden, indem die Verbindung der Formel (I) in der Modifikation I in Ethanol:Wasser (1:1) gelöst wird. Die Lösung wird bei einer Temperatur von etwa -20°C gelagert, bis das Lösungsmittel verdunstet ist. Man erhält so das Hydrat der Verbindung der Formel (I).

Das NMP-Solvat der Verbindung der Formel (I) kann hergestellt werden, indem die Verbindung der Formel (I) in der Modifikation I in 1-Methyl-2-Pyrrolidon suspendiert und bei Raumtemperatur gerührt wird. Nach 2 Tagen wird filtriert und das Produkt getrocknet. Man erhält so das NMP-Solvat der Verbindung der Formel (I) mit einem NMP-Anteil von 18,5 Gewichtsprozenten.

Die Einschlussverbindung mit THF der Verbindung der Formel (I) kann hergestellt werden, indem die Verbindung der Formel (I) in der Modifikation I in Tetrahydrofuran gelöst wird. Die Lösung wird bei Raumtemperatur gelagert, bis das Lösungsmittel verdunstet ist. Man erhält so die Einschlussverbindung mit THF der Verbindung der Formel (I).

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Ausführungsbeispiele

Die Thermogramme wurden unter Verwendung eines Differential Scanning Calorimeters DSC 7 bzw. Pyris-1 und Thermogravimetric Analyser TGA 7 der Fa. Perkin-Elmer erhalten. Die Röntgendiffraktogramme wurden in einem Stoe-Transmissionsdiffraktometer registriert. Die IR-, FIR-, NIR- und Raman-Spektren wurden mit Fourier-IR-Spektrometern IFS 66v (IR, FIR), IFS 28/N (NIR) und RFS 100 (Raman) der Fa. Bruker aufgenommen.

### Beispiel 1: 5-Chlor-N-({(5S)-2-oxo-3-[4-(3-oxo-4-morpholinyl)-phenyl]-1,3-oxazolidin-5-yl}-methyl)-2-thiol)hencarboxamid in der Modifikation I

Die Herstellung der Modifikation I der Titelverbindung ist in WO 01/47949 und WO 2004/060887 beschrieben.

### Beispiel 2: Herstellung von 5-Chlor-N-({(5S)-2-oxo-3-[4-(3-oxo-4-morpholinyl)-phenyl]-1,3-oxazolidin-5-yl}-methyl)-2-thiophencarboxamid in der Modifikation II

### Beispiel 2.1

208 g Chlorthiophencarbonsäure wurden in 1100 ml Toluol suspendiert und auf 75 bis 80°C erhitzt. Bei dieser Temperatur wurden innerhalb von 2 h 112 ml Thionylchlorid zugetropft. Die entstandene Reaktionslösung wurde bis zum Ende der Gasentwicklung weitere 2 h nachgerührt. Dabei wurde die Innentemperatur in 5°-Schritten auf 100-110°C erhöht. Das Gemisch wurde abgekühlt und die Lösung des Säurechlorids am Rotationsverdampfer eingeengt.

350 g Oxamin-Hydrochlorid wurden in 2450 ml NMP suspendiert, mit 385 ml Triethylamin versetzt und 15 min gerührt. Das Gemisch wurde auf 10°C gekühlt, mit der Lösung aus dem Säurechlorid und 70 ml Toluol versetzt und gerührt. Zu der Suspension wurden 350 ml Leitungswasser gegeben und auf 82°C erhitzt. Nach Filtration wurde der Wirkstoff mit 3,5 Wasser gefällt und 2 h nachgerührt. Trocknung bei 70°C im Vakuum.

### Beispiel 2.2

Ca. 200 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden in ca. 80 ml Tetrahydrofuran heiß gelöst. Die Lösung wurde filtriert und halbiert. Eine Hälfte wurde bei Raumtemperatur mit n-Heptan versetzt, bis der Wirkstoffausfiel. Der Rückstand wurde abfiltriert und bei Raumtemperatur getrocknet. Er wurde röntgendiffraktometrisch untersucht und entsprach der Titelverbindung in der Modifikation II.

### Beispiel 2.3

Ca. 200 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden in ca. 40 ml 1-Pentanol heiß gelöst. Die Lösung wurde filtriert und halbiert. Ein Hälfte wurde mit n-Heptan versetzt, bis der Wirkstoffausfiel. Der Rückstand wurde abfiltriert und bei Raumtemperatur getrocknet. Er wurde röntgendiffraktometrisch untersucht und entsprach der Titelverbindung in der Modifikation II.

### Beispiel 2.4

Ca. 200 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden in ca. 40 ml 1,4-Dioxan heiß gelöst. Die Lösung wurde filtriert und halbiert. Eine Hälfte wurde bei 50°C im Trockenschrank gelagert, bis das Lösungsmittel verdampft war. Der Rückstand wurde röntgendiffraktometrisch untersucht und entsprach der Titelverbindung in der Modifikation II.

### Beispiel 2.5

Ca. 50 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der amorphen Form, hergestellt durch Durchschmelzen auf der Kofler-Heizbank bei ca. 240°C und anschließende Schockkühlung auf Raumtemperatur, wurden in ca. 2 ml Ethanol suspendiert und 0,5 h bei 25°C gerührt. Das Kristallisat wurde isoliert und getrocknet. Der Rückstand wurde röntgendiffraktometrisch untersucht und entsprach der Titelverbindung in der Modifikation II.

### Beispiel 2.6

Ca. 100 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden in ca. 50 ml Aceton heiß gelöst. Die Lösung wurde filtriert und im Eisbad mit n-Heptan versetzt, bis der Wirkstoff ausfiel. Der Rückstand wurde abfiltriert und bei Raumtemperatur getrocknet. Er wurde röntgendiffraktometrisch untersucht und entsprach der Titelverbindung in der Modifikation II.

### Beispiel 3: Herstellung von 5-Chlor-N-({(5S)-2-oxo-3-[4-(3-oxo-4-morpholinyl)-phenyl]-1,3-oxazolidin-5-yl}-methyl)-2-thiophencarboxamid in der Modifikation III

Ca. 120 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden in ca. 50 ml Aceton heiß gelöst. Die Lösung wurde filtriert, mit ca. 50 ml Wasser versetzt und bei Raumtemperatuar stehengelassen, bis das Lösungsmittel verdunstet war. Der Rückstand wurde thermoanalytisch untersucht und entsprach der Titelverbindung in der Modifikation III.

### Beispiel 4: Herstellung des Hydrats von 5-Chlor-N-({(5S)-2-oxo-3-[4-(3-oxo-4-morpholinyl)-phenyl]-1,3-oxazolidin-5-yl}-methyl)-2-thiophencarboxamid

Ca. 400 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden in ca. 60 ml Ethanol:Wasser (1:1) heiß gelöst und filtriert. Ein Teil der Lösung wurde im Gefrierschrank bei einer Temperatur von etwa -20°C gelagert, bis das Lösungsmittel verdunstet war. Der Rückstand entsprach dem Hydrat der Titelverbindung.

### Beispiel 5: Herstellung des NMP-Solvates von 5-Chlor-N-({(5S)-2-oxo-3-[4-(3-oxo-4-morpholinyl)-phenyl]-1,3-oxazolidin-5-yl}-methyl)-2-thiophencarboxamid

Ca. 3,5 g von 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden in 10 ml 1-Methyl-2-pyrrolidon suspendiert und bei Raumtemperatur gerührt. Nach einigen Stunden wurden noch ca. 20 ml NMP zugegeben. Nach zwei Tagen wurde die Suspension abgesaugt und der Rückstand bei Raumtemperatur getrocknet. Der Rückstand wurde thermoanalytisch untersucht und entsprach dem NMP-Solvat der Titelverbindung mit einem NMP-Anteil von 18,5 Gewichtsprozenten.

### Beispiel 6: Herstellung der Einschlussverbindung mit THF von 5-Chlor-N-({(5S)-2-oxo-3-[4-(3-oxo-4-morpholinyl)-phenyl]-1,3-oxazolidin-5-yl}-methyl)-2-thiophencarboxamid

Ca. 400 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden in ca. 50 ml Tetrahydrofuran heiß gelöst und filtriert. Ein Teil der Lösung wurde bei Raumtemperatur gelagert, bis das Lösungsmittel verdunstet war. Der Rückstand wurdee thermoanalytisch untersucht und entsprach der Einschlussverbindung mit THF der Titelverbindung.

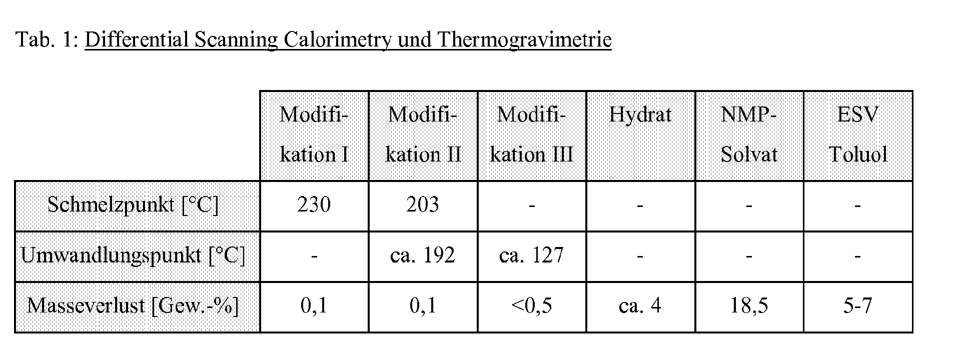

**Tab. 2: Röntgendiffraktometrie**

| Reflexe | | | | | |
|---|---|---|---|---|---|
| Modifikation I [2 Theta] | Modifikation II [2 Theta] | Modifikation III [2 Theta] | Hydrat [2 Theta] | NMP-Solvat [2 Theta] | ESV mit THF [2 Theta] |
| 8,9 | 12,8 | 11,7 | 3,6 | 4,8 | 9,0 |
| 12,0 | 17,7 | 16,5 | 14,3 | 5,8 | 12,0 |
| 14,3 | 18,1 | 17,5 | 16,4 | 7,3 | 14,3 |
| 16,5 | 18,4 | 19,1 | 16,6 | 10,9 | 14,7 |
| 17,4 | 19,0 | 19,6 | 17,5 | 14,5 | 16,5 |
| 18,1 | 19,9 | 19,8 | 19,3 | 15,2 | 16,8 |
| 19,5 | 20,8 | 23,1 | 19,6 | 15,7 | 17,5 |
| 19,9 | 21,6 | 23,2 | 19,9 | 16,0 | 19,6 |
| 21,7 | 22,1 | 23,8 | 20,2 | 17,6 | 19,9 |
| 22,5 | 22,9 | 24,3 | 21,7 | 17,9 | 21,7 |
| 23,4 | 24,1 | 28,1 | 22,5 | 20,0 | 22,5 |
| 24,1 | 26,1 | 28,2 | 24,2 | 20,6 | 23,4 |
| 24,5 | 26,4 | 31,2 | 25,6 | 21,3 | 24,5 |
| 24,7 | 26,6 | | 25,8 | 21,8 | 24,7 |
| 25,6 | 27,2 | | 28,8 | 22,3 | 25,2 |
| 26,4 | 27,5 | | 29,5 | 22,7 | 25,6 |
| 26,7 | 28,8 | | 31,8 | 23,1 | 26,4 |
| 30,0 | 29,8 | | 32,7 | 23,3 | 26,7 |
| 30,1 | 31,0 | | | 23,5 | 28,7 |
| 31,8 | 31,6 | | | 24,0 | 30,1 |
| | 32,9 | | | 24,7 | 31,0 |
| | | | | 24,9 | 31,8 |
| | | | | 25,2 | |
| | | | | 26,0 | |
| | | | | 26,5 | |
| | | | | 26,9 | |
| | | | | 28,0 | |
| | | | | 28,8 | |
| | | | | 29,2 | |
| | | | | 29,5 | |
| | | | | 29,8 | |

**Tab. 3: IR-Spektroskopie**

| | Peakmaxima | | | |
|---|---|---|---|---|
| Modifikation II [cm⁻¹]] | Modifikation II [cm⁻¹] | Modifikatio III [cm⁻¹] | Hydrat [cm⁻¹] | NMP-Solvat [cm⁻¹] |
| 564 | 552 | 515 | 708 | 497 |
| 686 | 598 | 546 | 755 | 547 |
| 708 | 692 | 596 | 776 | 562 |
| 746 | 713 | 611 | 820 | 708 |
| 757 | 725 | 644 | 920 | 749 |
| 830 | 756 | 688 | 992 | 819 |
| 846 | 809 | 709 | 1054 | 838 |
| 920 | 825 | 748 | 1089 | 921 |
| 991 | 833 | 755 | 1120 | 987 |
| 1011 | 924 | 776 | 1146 | 1065 |
| 1056 | 994 | 812 | 1221 | 1088 |
| 1077 | 1067 | 816 | 1289 | 1123 |
| 1120 | 1085 | 842 | 1312 | 1143 |
| 1146 | 1097 | 864 | 1324 | 1162 |
| 1163 | 1121 | 921 | 1340 | 1225 |
| 1219 | 1146 | 992 | 1349 | 1242 |
| 1286 | 1232 | 1016 | 1413 | 1260 |
| 1307 | 1285 | 1054 | 1429 | 1292 |
| 1323 | 1310 | 1089 | 1469 | 1302 |
| 1341 | 1328 | 1121 | 1485 | 1315 |
| 1374 | 1345 | 1148 | 1518 | 1330 |
| 1411 | 1415 | 1161 | 1555 | 1354 |
| 1429 | 1431 | 1224 | 1630 | 1387 |
| 1470 | 1473 | 1261 | 1668 | 1414 |
| 1486 | 1523 | 1288 | 1738 | 1421 |
| 1517 | 1554 | 1313 | 2873 | 1430 |
| 1546 | 1631 | 1325 | 3341 | 1471 |
| 1605 | 1648 | 1348 | | 1517 |
| 1646 | 1663 | 1380 | | 1566 |
| 1669 | 1723 | 1412 | | 1636 |
| 1737 | 1745 | 1429 | | 1665 |
| 2867 | 3341 | 1473 | | 1755 |
| 2895 | | 1518 | | 2887 |
| 2936 | | 1553 | | 2928 |
| 2976 | | 1629 | | 2948 |
| 3354 | | 1668 | | 2983 |
| | | 1741 | | 3045 |
| | | 2878 | | 3085 |
| | | 3080 | | 3247 |
| | | 3340 | | |

**Tab. 4: Raman-Spektroskopie**

| | Peakmaxima | | | |
|---|---|---|---|---|
| Modifikation I [cm⁻¹] | Modifikation II [cm⁻¹] | Modifikation III [cm⁻¹] | Hydrat [cm⁻¹] | NMP-Solvat [cm⁻¹] |
| 84 | 86 | 85 | 85 | 85 |
| 111 | 184 | 112 | 111 | 105 |
| 642 | 276 | 165 | 132 | 119 |
| 672 | 345 | 671 | 642 | 485 |
| 687 | 485 | 712 | 672 | 671 |
| 745 | 643 | 743 | 711 | 710 |
| 779 | 672 | 778 | 744 | 743 |
| 792 | 716 | 793 | 778 | 776 |
| 1083 | 742 | 996 | 793 | 800 |
| 1099 | 778 | 1093 | 922 | 1193 |
| 1232 | 800 | 1288 | 1073 | 1229 |
| 1280 | 864 | 1322 | 1083 | 1233 |
| 1307 | 925 | 1428 | 1097 | 1242 |
| 1325 | 995 | 1442 | 1231 | 1259 |
| 1343 | 1086 | 1475 | 1301 | 1282 |
| 1428 | 1119 | 1555 | 1325 | 1313 |
| 1473 | 1149 | 1610 | 1428 | 1319 |
| 1485 | 1196 | 1626 | 1473 | 1328 |
| 1548 | 1227 | 1663 | 1485 | 1412 |
| 1605 | 1248 | 1669 | 1548 | 1433 |
| 1638 | 1282 | 1723 | 1605 | 1473 |
| 1664 | 1310 | 2881 | 1638 | 1608 |
| 1722 | 1330 | 2992 | 1722 | 1629 |
| 2899 | 1432 | 3020 | 2885 | 1660 |
| 2944 | 1474 | 3098 | 2898 | 1763 |
| 2983 | 1556 | | 2944 | 2844 |
| 3074 | 1608 | | 2983 | 2889 |
| | 1631 | | 3074 | 2931 |
| | 1648 | | | 2946 |
| | 1722 | | | 2984 |
| | 2885 | | | 3075 |
| | 2938 | | | 3096 |
| | 2989 | | | |
| | 3077 | | | |
| | 3091 | | | |

**Tab. 5: FIR-Spektroskopie**

| | Peakmaxima | | |
|---|---|---|---|
| Modifikation I [cm⁻¹] | Modifikation II [cm⁻¹] | Hydrat [cm⁻¹] | NMP-Solvat [cm⁻¹] |
| 82 | 83 | 83 | 84 |
| 97 | 96 | 96 | 126 |
| 138 | 126 | 126 | 137 |
| 169 | 146 | 134 | 169 |
| 179 | 159 | 138 | 190 |
| 210 | 190 | 156 | 209 |
| 226 | 213 | 168 | 237 |
| 247 | 244 | 179 | 282 |
| 272 | 279 | 226 | 297 |
| 283 | 293 | 247 | 308 |
| 298 | 304 | 271 | 317 |
| 303 | 344 | 298 | 344 |
| 350 | 363 | 304 | 353 |
| 394 | 401 | 349 | 400 |
| 417 | 416 | 394 | 413 |
| 438 | 437 | 408 | 417 |
| 458 | 456 | 417 | 432 |
| 475 | 484 | 438 | 459 |
| 484 | | 455 | 471 |
| | | 472 | 485 |
| | | 484 | 498 |

**Tab. 6: NIR-Spektroskopie**

| | Peakmaxima | | | |
|---|---|---|---|---|
| Modifikation I [cm⁻¹] | Modifikation I [cm⁻¹] | Modifikation I [cm⁻¹] | Hydrat [cm⁻¹] | NMP-Solvat [cm⁻¹] |
| 4082 | 4086 | 4080 | 4083 | 4040 |
| 4142 | 4228 | 4218 | 4228 | 4084 |
| 4170 | 4418 | 4329 | 4305 | 4213 |
| 4228 | 4457 | 4398 | 4384 | 4382 |
| 4299 | 4634 | 4606 | 4631 | 4552 |
| 4376 | 4905 | 4891 | 4905 | 4638 |
| 4429 | 5846 | 5066 | 5145 | 4830 |
| 4479 | 5911 | 6022 | 5760 | 5815 |
| 4633 | 6026 | 6072 | 5833 | 6091 |
| 4791 | 6081 | | 5889 | 7213 |
| 4877 | 6582 | | 6023 | 8527 |
| 4907 | | | 6076 | |
| 5081 | | | 6555 | |
| 5760 | | | 6868 | |
| 5885 | | | | |
| 6002 | | | | |
| 6441 | | | | |
| 6564 | | | | |
| 8473 | | | | |
| 8833 | | | | |

### Beispiel 7: Herstellung von 5-Chlor-N-({(5S)-2-oxo-3-[4-(3-oxo-4-morpholinyl)-phenyl]-1,3-oxazolidin-5-yl}-methyl)-2-thiophencarboxamid in amorpher Form

### Beispiel 7.1

Ca. 50 mg 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden auf der Kofler-Heizbank bei ca. 240°C durchgeschmolzen und anschließend durch Schockkühlung auf Raumtemperatur gebracht. Der Wirkstoff wurde röntgendiffraktometrisch untersucht und lag in der amorphen Form vor.

### Beispiel 7.2

Ca. 3 g 5-Chlor-N-({(5*S*)-2-oxo-3-[4-(3-oxo-4-morpholinyl)-phenyl]-1,3-oxazolidin-5-yl}-methyl)-2-thiophencarboxamid in der Modifikation I wurden im Trockenschrank bei ca. 250°C durchgeschmolzen und anschließend durch Schockkühlung auf Raumtemperatur gebracht. Der Wirkstoff wurde röntgendiffraktometrisch untersucht und lag in der amorphen Form vor.

Tab. 7: Differential Scanning Calorimetry und Thermogravimetrie (amorphe Form)
Glasumwandlungstemperatur: ca.83°C

**Tab. 8: Spektroskopie (amorphe Form)**

| | Peakmaxima | | |
|---|---|---|---|
| IR [cm⁻¹] | Raman [cm⁻¹] | FIR [cm⁻¹] | NIR [cm⁻¹] |
| 467 | 486 | 91 | 4006 |
| 512 | 642 | 97 | 4081 |
| 550 | 673 | 137 | 4224 |
| 595 | 711 | 169 | 4307 |
| 613 | 742 | 246 | 4403 |
| 643 | 781 | 272 | 4634 |
| 689 | 923 | 297 | 4875 |
| 709 | 965 | 248 | 5193 |
| 725 | 1016 | 393 | 5865 |
| 750 | 1078 | 416 | 6017 |
| 810 | 1126 | 438 | 6073 |
| 834 | 1224 | 456 | 6696 |
| 864 | 1243 | 474 | 7028 |
| 921 | 1290 | 474 | 8452 |
| 995 | 1326 | | 8873 |
| 1015 | 1428 | | |
| 1026 | 1479 | | |
| 1058 | 1548 | | |
| 1083 | 1607 | | |
| 1126 | 1642 | | |
| 1161 | 2158 | | |
| 1222 | 2975 | | |
| 1288 | 3090 | | |
| 1312 | | | |
| 1325 | | | |
| 1380 | | | |
| 1407 | | | |
| 1428 | | | |
| 1480 | | | |
| 1516 | | | |
| 1549 | | | |
| 1607 | | | |
| 1647 | | | |
| 1753 | | | |
| 2126 | | | |
| 2869 | | | |
| 2933 | | | |
| 2967 | | | |
| 3084 | | | |
| 3317 | | | |

## Patentansprüche

1. Verbindung der Formel (I) 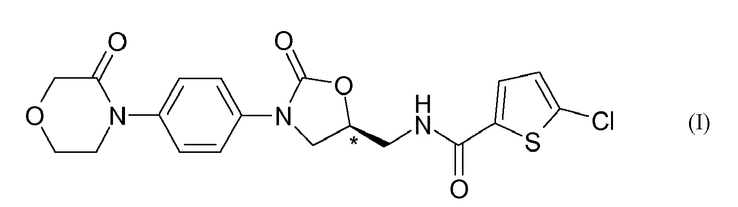 in der Modifikation II oder in der amorphen Form.

2. Verfahren zur Herstellung der Verbindung der Formel (I) in der Modifikation II, **dadurch gekennzeichnet, dass** die Verbindung der Formel (I) in der Modifikation I in einem inerten Lösungsmittel gelöst und die Verbindung durch Zugabe eines Fällungsmittels gefällt wird.

3. Verfahren zur Herstellung der Verbindung der Formel (I) in der Modifikation II, **dadurch gekennzeichnet, dass** die Verbindung der Formel (I) in der Modifikation I in einem inerten Lösungsmittel gelöst und die Lösung bei erhöhter Temperatur bis zum vollständigen Verdampfen des Lösungsmittels gelagert wird.

4. Verfahren zur Herstellung der Verbindung der Formel (I) in der Modifikation II, **dadurch gekennzeichnet, dass** die Verbindung der Formel (I) in der amorphen Form in einem wasserfreien inerten Lösungsmittel suspendiert und die Suspension bis zur quantitativen Umwandlung in die Modifikation II gerührt oder geschüttelt wird.

5. Verfahren zur Herstellung der Verbindung der Formel (I) in der amorphen Form, **dadurch gekennzeichnet, dass** man die Verbindung der Formel (I) in einer kristallinen Form vollständig durchschmilzt und anschließend schnell abkühlt.

6. Verbindung der Formel (I) in der amorphen Form erhältlich, indem man die Verbindung der Formel (I) in einer kristallinen Form vollständig durchschmilzt und schnell abkühlt.

7. Verbindung der Formel (I) in der Modifikation II oder in der amorphen Form zur Behandlung und/oder Prophylaxe von Krankheiten.

8. Verwendung der Verbindung der Formel (I) in der Modifikation II oder in der amorphen Form zur Herstellung eines Arzneimittels zur Behandlung und/oder Prophylaxe von thromboembolischen Erkrankungen.

9. Verwendung der Verbindung der Formel (I) in der Modifikation II oder in der amorphen Form zur Verhinderung der Blutkoagulation in vitro.

10. Arzneimittel enthaltend die Verbindung der Formel (I) in der Modifikation II oder in der amorphen Form in Kombination mit einem inerten, nicht-toxischen, pharmazeutisch geeigneten Hilfsstoff.

11. Arzneimittel enthaltend die Verbindung der Formel (I) in der Modifikation II oder in der amorphen Form in Kombination mit einem weiteren Wirkstoff.

12. Arzneimittel nach Anspruch 10 oder 11 zur Behandlung und/oder Prophylaxe von thromboembolischen Erkrankungen.

13. Verfahren zur Behandlung und/oder Prophylaxe von thromboembolischen Erkrankungen bei Menschen und Tieren unter Verwendung einer antikoagulatorisch wirksamen Menge der Verbindung der Formel (I) in der Modifikation II oder in der amorphen Form oder eines Arzneimittels, wie in einem der Ansprüche 10 bis 12 definiert.

14. Verfahren zur Verhinderung der Blutkoagulation in vitro, **dadurch gekennzeichnet, dass** eine antikoagulatorisch wirksame Menge der Verbindung der Formel (I) in der Modifikation II oder in der amorphen Form zugegeben wird.
